# EUROPÄISCHE PATENTSCHRIFT

(11) **EP 3 400 870 B1**
(45) Veröffentlichungstag und Bekanntmachung des Hinweises auf die Patenterteilung: **30.10.2019**
(21) Anmeldenummer: 17170786.2
(22) Anmeldetag: 12.05.2017
(51) Int. Cl.: A61B 5/055, A61G 7/05

(54) **VORRICHTUNG UND VERFAHREN ZU EINEM STEUERN EINER PATIENTENLAGERUNGSVORRICHTUNG ANHAND EINES UMFELDPARAMETERS MITTELS EINER STEUERUNGSEINHEIT**
DEVICE AND METHOD FOR CONTROLLING A PATIENT SUPPORTING DEVICE USING AN ENVIRONMENTAL PARAMETER BY MEANS OF A CONTROL UNIT
DISPOSITIF ET PROCÉDÉ DE COMMANDE D'UN DISPOSITIF DE POSITIONNEMENT DE PATIENT AU MOYEN D'UN PARAMÈTRE D'ENVIRONNEMENT À L'AIDE D'UNE UNITÉ DE COMMANDE

(43) Veröffentlichungstag der Anmeldung: 14.11.2018
(73) Patentinhaber: Siemens Healthcare GmbH, 91052 Erlangen (DE)
(72) Erfinder: Biber, Stephan, 91056 Erlangen (DE)

(56) Entgegenhaltungen:
- DE-A1-102006 032 798
- DE-A1-102013 208 610
- DE-A1-102013 213 213
- US-A1- 2011 154 569

## Beschreibung

Die vorliegende Erfindung betrifft eine Patientenlagerungsvorrichtung mit einem Liegentisch, einer Fahreinheit, die zu einer Bewegung der Patientenlagerungsvorrichtung ausgelegt ist, einer Steuerungseinheit und einer Sensoreinheit. Des Weiteren betrifft die vorliegende Erfindung eine Magnetresonanzvorrichtung mit einer Patientenlagerungsvorrichtung sowie ein Verfahren zu einer Steuerung einer Fahreinheit einer Patientenlagerungsvorrichtung mittels einer Steuerungseinheit.

Andockbare Patientenlagerungsvorrichtungen müssen für eine Verwendung mit einer Magnetresonanzvorrichtung zunächst an die Magnetresonanzvorrichtung angedockt werden. Der Vorgang des Andockens der Patientenlagerungsvorrichtung erfolgt jedoch bisher weitgehend manuell durch einen Benutzer, beispielsweise ein medizinisches Bedienpersonal. Der Benutzer muss hierbei die Patientenlagerungsvorrichtung zur Magnetresonanzvorrichtung fahren und/oder schieben und dabei möglichst exakt auf eine Andockeinheit der Magnetresonanzvorrichtung treffen. Dies kann jedoch je nach Übung des Benutzers einige Zeit in Anspruch nehmen.

Eine andockbar Patientenlagerungsvorrichtung ist beispielsweise aus DE 10 2014 212 202, DE 10 2013 213 213, DE 10 2013 208 610 und aus DE 10 2012 217 634 bekannt.

Der vorliegenden Erfindung liegt insbesondere die Aufgabe zugrunde, eine zumindest teilweise selbsttätiges Fahren und/oder Andocken einer Patientenlagerungsvorrichtung zu ermöglichen. Die Aufgabe wird durch die Merkmale der unabhängigen Ansprüche gelöst. Vorteilhafte Ausgestaltungen sind in den Unteransprüchen beschrieben.

Die Erfindung geht aus von einer Patientenlagerungsvorrichtung mit einem Liegentisch, einer Fahreinheit, die zu einer Bewegung der Patientenlagerungsvorrichtung ausgelegt ist, einer Steuerungseinheit und einer Sensoreinheit.

Es wird vorgeschlagen, dass die Sensoreinheit zumindest einen Umfeldparameter der Patientenlagerungsvorrichtung erfasst und Steuerungseinheit in Abhängigkeit des zumindest einen Umfeldparameters einen Steuerparameter zur Steuerung der Fahreinheit ermittelt.

Die Patientenlagerungsvorrichtung ist von einer an eine Magnetresonanzvorrichtung andockbare Patientenlagerungsvorrichtung gebildet. Hierzu weist die Patientenlagerungsvorrichtung zusätzlich eine Andockeinheit auf, die in Richtung einer Bewegungsrichtung an einer Frontseite der Patientenlagerungsvorrichtung angeordnet ist. Die Andockeinheit der Patientenlagerungsvorrichtung ist bevorzugt korrespondierend und/oder zu einer Andockeinheit der Magnetresonanzvorrichtung ausgebildet. An einer Heckseite der Patientenlagerungsvorrichtung weist die Patientenlagerungsvorrichtung vorzugsweise eine Bedieneinheit, insbesondere eine Griffeinheit, auf, mittels der ein Benutzer einer Kraft zum Bewegen der Patientenlagerungsvorrichtung auf die Patientenlagerungsvorrichtung übertragen kann.

Der Liegentisch ist bevorzugt zu einer Lagerung des Patienten ausgelegt. Der Liegentisch ist zudem in horizontaler Richtung, insbesondere in Richtung einer Längsrichtung des Liegentischs verfahrbar, so dass der Liegentisch nach einem Andocken der Patientenlagerungsvorrichtung an eine Magnetresonanzvorrichtung in einen Patientenaufnahmebereich der Magnetresonanzvorrichtung einbringbar und/oder einfahrbar ist. Zudem kann der Liegentisch auch in vertikaler Richtung, insbesondere in einer Höhe, bewegbar ausgestaltet sein. Dies kann beispielsweise ein Positionieren eines Patienten auf dem Liegentisch erleichtern.

Die Fahreinheit weist bevorzugt mehrere Rollelemente und/oder Rollkörper auf, wie beispielsweise Räder, mittels denen die Patientenlagerungsvorrichtung bewegt, insbesondere verfahren, werden kann. Vorzugsweise weist die Fahreinheit vier oder fünf Rollelemente und/oder Rollkörper auf. Zudem kann die Fahreinheit auch eine Antriebseinheit umfassen, die zu einem Antrieb der Rollelemente während eines Fahrens der Patientenlagerungsvorrichtung vorgesehen ist. Mittels der Fahreinheit ist die Patientenlagerungsvorrichtung in einem abgedockten Zustand bewegbar ausgestaltet. In dem abgedockten Zustand ist die Patientenlagerungsvorrichtung nicht an die Magnetresonanzvorrichtung angedockt.

Die Sensoreinheit erfasst einen Umfeldparameter der Patientenlagerungsvorrichtung. Der Umfeldparameter umfasst vorteilhaft eine Magnetfeldstärke und/oder einen Wert eines von einem supraleitenden Hauptmagneten der Magnetresonanzvorrichtung erzeugten B₀-Feldes am Ort der Patientenlagerungsvorrichtung. Somit wird mittels der Sensoreinheit eine Magnetfeldstärke eines statischen Magnetfelds des supraleitenden Hauptmagneten der Magnetresonanzvorrichtung am Ort der Patientenlagerungsvorrichtung erfasst. Das statische Magnetfeld ist bevorzugt in einem Isozentrum und/oder einem Untersuchungsbereich der Magnetresonanzvorrichtung nahezu homogen. Außerhalb des Isozentrums und/oder des Untersuchungsbereichs variiert jedoch das Magnetfeld stark in Abhängigkeit von einer Position, insbesondere in Abhängigkeit eines Abstands zu dem Isozentrum und/oder dem Untersuchungsbereich der Magnetresonanzvorrichtung. Befindet sich beispielsweise die Patientenlagerungsvorrichtung in der Nähe zu einer Magnetresonanzvorrichtung, kann somit ein Umfeldparameter mit einer hohen Magnetfeldstärke mittels der Sensoreinheit erfasst werden. Befindet sich die Patientenlagerungsvorrichtung dagegen weit entfernt zur Magnetresonanzvorrichtung, insbesondere außerhalb einer Reichweite eines von der Magnetresonanzvorrichtung erzeugten Magnetfelds, kann ein Umfeldparameter mit einer geringen und/oder nicht vorhandenen Magnetfeldstärke mittels der Sensoreinheit erfasst werden.

Alternativ oder zusätzlich kann der Umfeldparameter auch einen Wert eines B₁-Feldes der Magnetresonanzvorrichtung am Ort der Patientenlagerungsvorrichtung umfassen, wobei der Wert des B₁-Feldes mittels der Sensoreinheit erfassbar ist. Des Weiteren kann der Umfeldparameter auch einen Wert eines Gradientenfelds der Magnetresonanzvorrichtung am Ort der Patientenlagerungsvorrichtung umfassen, wobei der Wert des Gradientenfelds mittels der Sensoreinheit erfassbar ist. Zudem kann es auch vorgesehen sein, dass der Umfeldparameter einen Wert und/oder eine Anzahl an Lichtmarkierungen, die vom Magnetresonanzsystem ausgesendet werden, am Ort der Patientenlagerungsvorrichtung umfassen, wobei die Anzahl der Lichtmarkierungen mittels der Sensoreinheit erfassbar ist.

Der Umfeldparameter, der beispielsweise einen Wert einer Magnetfeldstärke umfasst, kann dabei besonders vorteilhaft eine Positionsinformation umfassen, wobei die Positionsinformation abhängig ist von dem erfassten Wert der Magnetfeldstärke und/oder dem erfassten Wert des B₀-Feldes. Sofern der Umfeldparameter einen Wert eines B₁-Feldes oder einen Wert eines Gradientenfelds oder eine Anzahl an Lichtmarkierungen, kann eine Positionsinformation in Abhängigkeit des B₁-Feldes oder des Gradientenfelds oder der Anzahl an Lichtmarkierungen ermittelt und/oder generiert werden. Die Positionsinformation umfasst dabei bevorzugt eine Position der Patientenlagerungsvorrichtung und/oder der Sensoreinheit der Patientenlagerungsvorrichtung bezüglich der Magnetresonanzvorrichtung. Beispielsweise umfasst die Steuerungseinheit bevorzugt mehrere Feldkarten von unterschiedlichen Magneten, so dass anhand eines Vergleichs der erfassten Umfeldparameter mit den Feldkarten ein Magnettyp der Magnetresonanzvorrichtung ermittelt werden kann. Anhand der ausgewählten Feldkarte kann zudem auch besonders einfach und exakt die Position der Patientenlagerungsvorrichtung bezüglich der Magnetresonanzvorrichtung ermittelt werden.

Die Steuerungseinheit weist bevorzugt eine Recheneinheit mit zumindest einem Prozessor auf. Des Weiteren umfasst die Steuerungseinheit bevorzugt Steuerungsprogramme und/oder Steuerungssoftware zu einer Steuerung der Patientenlagerungsvorrichtung. Beispielsweise kann die Steuerung zumindest teilweise eine Steuerung für eine Bewegung des Liegentischs in horizontaler und/oder in vertikaler Richtung umfassen. Zudem kann die Steuerung zumindest teilweise auch eine Steuerung der Fahreinheit, insbesondere der Antriebseinheit der Fahreinheit, umfassen. Weiterhin kann die Steuerung auch eine Steuerung einer Bewegungsrichtung der Patientenlagerungsvorrichtung umfassen. Vorzugweise umfasst der Steuerparameter zur Steuerung der Fahreinheit einen Parameter zur Ansteuerung der Antriebseinheit der Fahreinheit und/oder einen Parameter zur Einstellung einer Bewegungsrichtung an den Rollelementen der Fahreinheit.

Zur Bestimmung einer Position in Abhängigkeit von dem Umfeldparameter, insbesondere einem Wert einer Magnetfeldstärke, wird auf DE 10 2009 021 026 verwiesen.

Die Erfindung weist den Vorteil auf, dass der Benutzer eine vorteilhafte Unterstützung zumindest während eines Andockvorgangs der Patientenlagerungsvorrichtung an die Magnetresonanzvorrichtung mittels einer Steuerung der Fahreinheit anhand des Steuerparameters durch die Steuerungseinheit erhalten kann. Insbesondere kann derart die Patientenlagerungsvorrichtung zumindest teilweise anhand von Steuerparametern gesteuert werden, wenn die Patientenlagerungsvorrichtung in einer Nähe zu der Magnetresonanzvorrichtung angeordnet ist, da dann ein aussagekräftiger Umfeldparameter erfasst werden kann und/oder vorliegt, der sich auch mit einer Positionsänderung der Patientenlagerungsvorrichtung bezüglich der Magnetresonanzvorrichtung ändert. Befindet sich dagegen die Patientenlagerungsvorrichtung mit sehr großem Abstand zu der Magnetresonanzvorrichtung, machen sich Änderungen des Abstands und/oder eine Positionsänderung der Patientenlagerungsvorrichtung bezüglich der Magnetresonanzvorrichtung kaum in einem erfassten Umfeldparameter bemerkbar.

Laut Erfindung wird vorgeschlagen, dass die Sensoreinheit zumindest einen Umfeldsensor aufweist. Zur Erfassung eines B₀-Feldes weist der zumindest eine Umfeldsensor einen B₀-Feldsensor auf. Vorzugsweise umfasst hierbei der zumindest eine B₀-Feldsensor einen Hallsensor. Eine derartig ausgebildete und/oder ausgestaltete Sensoreinheit weist den Vorteil auf, dass eine besonders kostengünstige Sensoreinheit für die Erfassung des zumindest einen Umfeldparameters zur Verfügung steht. Der B₀-Feldsensor kann dabei einen 1D-Feldsensor umfassen. Bevorzugterweise umfasst der B₀-Feldsensor einen 2D-Feldsensor. Besonders vorteilhaft jedoch umfasst der B₀-Feldsensor einen 3D-Feldsensor. Der resultierende Umfeldparameter, der mittels des B₀-Feldsensors erfasst wird, kann dabei den Betrag des statischen Magnetfeldes betreffen, beispielsweise durch Kombination mehrerer in unterschiedlichen, nicht-parallelen Ebenen angeordneten Hallsensoren, sodass die Flächennormalen der Ebenen einen 3-dimensionalen Raum aufspannen. Denkbar ist aber auch, dass der erfasste Umfeldparameter aber auch die Stärke einer Komponente in einer vorbestimmten Richtung umfasst. Die Richtung kann dabei durch die Anordnung des B₀-Feldsensors an der Patientenlagerungsvorrichtung definiert sein.

In einer alternativen Ausgestaltung der Erfindung kann die Sensoreinheit auch zumindest einen B₀-Feldsensor aufweisen, der eine von einem Hallsensor abweichende Ausbildung aufweist. Zudem kann in einer alternativen Ausgestaltung der Erfindung der zumindest eine Umfeldsensor auch einen Magnetfeldsensor zur Erfassung eines B1-Feldes und/oder eines Gradientenfeldes oder auch einen Lichtsensor aufweisen.

Laut Erfindung weist die Patientenlagerungsvorrichtung einen Frontbereich mit einer Andockeinheit auf, wobei die Andockeinheit zu einem Andocken an eine Magnetresonanzvorrichtung ausgelegt ist, wobei die Sensoreinheit in dem Frontbereich angeordnet ist. Hierdurch kann vorteilhaft für denjenigen Bereich der Patientenlagerungsvorrichtung, für den aufgrund eines Andockens an die Magnetresonanzvorrichtung eine besonders exakte Positionierung erforderlich ist, eine Positionsinformation anhand des Umfeldparameters bereitgestellt und/oder ermittelt werden.

Des Weiteren kann es vorgesehen sein, dass die Sensoreinheit zumindest zwei Umfeldsensoren aufweist, die beabstandet zueinander angeordnet sind. Dies ermöglicht eine besonders exakte und genaue Erfassung des Umfeldparameters mittels der zumindest zwei Umfeldsensoren. Insbesondere kann hierbei eine hohe Genauigkeit und/oder Eindeutigkeit der erfassten Messwerte mittels der zumindest zwei Umfeldsensoren erreicht werden. Unter beabstandet angeordnet soll hierbei insbesondere verstanden werden, dass die zumindest zwei Umfeldsensoren verteilt an der Patientenlagerungsvorrichtung angeordnet sind und/oder mit Abstand zueinander an der Patientenlagerungsvorrichtung angeordnet sind.

In einer weiteren vorteilhaften Ausgestaltung der Erfindung kann es vorgesehen sein, dass die Sensoreinheit zumindest einen Umfeldsensor aufweist, der innerhalb des Liegentischs angeordnet ist. Hierdurch kann gewährleistet werden, dass der zumindest eine Umfeldsensor, insbesondere der zumindest eine B₀-Feldsensor, einen maximalen Abstand zu einem Boden und/oder Untergrund, auf dem die Patientenlagerungsvorrichtung verfahrbar angeordnet ist, aufweist. Ein derartiger Boden und/oder Untergrund kann beispielsweise eine Bewehrung aus Eisen umfassen, die zu einer lokalen B₀-Verzerrung führen kann und somit zu gefälschten Ergebnissen führen kann.

Darüber hinaus kann es vorgesehen sein, dass die Patientenlagerungsvorrichtung eine Benutzerschnittstelle aufweist, die zu einem Einstellen eines Schwellwerts des Umfeldparameters ausgebildet ist, wobei der Schwellwert ein Kriterium für den Umfeldparameter zur Ermittlung des Steuerparameters zur Steuerung der Fahreinheit mittels der Steuerungseinheit festlegt. Vorzugsweise erfolgt die Eingabe des Schwellwerts des Umfeldparameters manuell durch einen Benutzer. Mittels des Schwellwerts kann vorteilhaft eine Sicherheit bei einem Transport der Patientenlagerungsvorrichtung erhöht werden, da beispielsweise eine automatische Steuerung der Fahreinheit der Patientenlagerungsvorrichtung erst ab einem Erreichen und/oder Überschreiten des Schwellwerts durch den erfassten Umfeldparameter erfolgen kann. Somit kann beispielsweise verhindert werden, dass die Steuerungseinheit bereits die Fahreinheit ansteuert, auch wenn die Patientenlagerungsvorrichtung noch außerhalb eines Untersuchungsraums ist und sich somit zwischen der Patientenlagerungsvorrichtung und der Magnetresonanzvorrichtung eine Wand befindet. Dabei kann es auch vorgesehen sein, dass bereits eine Untergrenze des Schwellwerts vorgegeben ist, und diese Untergrenze als Schwellwert vorbelegt ist. Für den Benutzer könnte demnach bei einer Eingabe des Schwellwerts somit den Schwellwert auf einen gewünschten Wert hochsetzen.

In einer vorteilhaften Weiterbildung der Erfindung kann es vorgesehen sein, dass die Steuerungseinheit eine Kommunikationsschnittstelle für eine Kommunikation mit einer Magnetresonanzvorrichtung aufweist. Vorzugsweise umfasst die Kommunikationsschnittstelle eine Datenschnittstelle, insbesondere eine kabellose und/oder drahtlose Datenschnittstelle, wie beispielswiese eine Funkschnittstelle. Mittels der Kommunikationsschnittstelle können vorteilhaft Informationen zwischen der Steuerungseinheit der Patientenlagerungsvorrichtung und der Magnetresonanzvorrichtung, beispielsweise einer Systemsteuereinheit der Magnetresonanzvorrichtung, ausgetauscht werden. Beispielsweise kann derart eine Information über eine Bauart eines Magneten und/oder über ein Streufeld des Magneten der Magnetresonanzvorrichtung an die Steuerungseinheit der Patientenlagerungsvorrichtung übertragen werden. Durch den Informationsaustausch kann eine besonders schnelle Positionsermittlung der Patientenlagerungsvorrichtung bezüglich der Magnetresonanzvorrichtung erreicht werden, da bereits durch Informationsübermittlung eine magnettypische Feldkarte innerhalb der Steuerungseinheit ausgewählt wurde und/oder ein magnettypisches Streufeld innerhalb der Steuerungseinheit zur Verfügung steht, anhand dessen eine Pensionierung der Patientenlagerungsvorrichtung in Abhängigkeit von dem erfassten Umfeldparameter erfolgen kann.

Weiterhin geht die Erfindung aus von einer Magnetresonanzvorrichtung mit einer Patientenlagerungsvorrichtung, wobei die Patientenlagerungsvorrichtung einen Liegentisch, eine Fahreinheit, die zu einer Bewegung der Patientenlagerungsvorrichtung ausgelegt ist, einer Steuerungseinheit und einer Sensoreinheit aufweist, wobei die Sensoreinheit zumindest einen Umfeldparameter der Patientenlagerungsvorrichtung erfasst und die Steuerungseinheit in Abhängigkeit des zumindest einen Umfeldparameters einen Steuerparameter zur Steuerung der Fahreinheit ermittelt.

Bevorzugt weist die Magnetresonanzvorrichtung eine Andockeinheit, die zu einem Andocken der Patientenlagerungsvorrichtung an die Magnetresonanzvorrichtung ausgelegt ist. Die Andockeinheit ist bevorzugt korrespondierend zu einer Andockeinheit der Patientenlagerungsvorrichtung ausgebildet. Des Weiteren weist die Magnetresonanzvorrichtung einen Patientenaufnahmebereich auf, der zu einer Aufnahme eines Patienten während einer Magnetresonanzuntersuchung ausgelegt ist. Der Patient wird in den Patientenaufnahmebereich mittels der Patientenlagerungsvorrichtung, insbesondere mittels des innerhalb des Patientenaufnahmebereichs bewegbaren Liegentischs der Patientenlagerungsvorrichtung, eingebracht. Der Patientenaufnahmebereich ist bevorzugt von einer Magneteinheit, die eine supraleitenden Hauptmagneten, eine Gradientenspuleneinheit und eine Hochfrequenzantenneneinheit aufweist, zylinderförmig umgeben.

Die Vorteile der erfindungsgemäßen Magnetresonanzvorrichtung entsprechen im Wesentlichen den Vorteilen der erfindungsgemäßen Patientenlagerungsvorrichtung, welche vorab im Detail ausgeführt sind. Hierbei erwähnte Merkmale, Vorteile oder alternative Ausführungsformen können ebenso auch auf die anderen beanspruchten Gegenstände übertragen werden und umgekehrt.

Des Weiteren geht die Erfindung aus von einem Verfahren zu einer Steuerung einer Fahreinheit einer Patientenlagerungsvorrichtung mittels einer Steuerungseinheit, umfassend die folgenden Schritte:
- Erfassen von zumindest einem Umfeldparameter der Patientenlagerungsvorrichtung mittels einer Sensoreinheit,
- Ermitteln von zumindest einem Steuerparameter für eine Ansteuerung der Fahreinheit mittels der Steuerungseinheit, wobei die Ermittlung des zumindest einen Steuerparameters in Abhängigkeit des zumindest einen Umfeldparameters erfolgt, und
- Ansteuerung der Fahreinheit gemäß dem ermittelten Steuerparameter mittels der Steuerungseinheit.

Die Erfindung weist den Vorteil auf, dass der Benutzer eine vorteilhafte Unterstützung zumindest während eines Andockvorgangs der Patientenlagerungsvorrichtung an die Magnetresonanzvorrichtung mittels einer Steuerung der Fahreinheit anhand des Steuerparameters durch die Steuerungseinheit erhalten kann. Insbesondere kann derart die Patientenlagerungsvorrichtung zumindest teilweise anhand von Steuerparametern gesteuert werden, wenn die Patientenlagerungsvorrichtung in einer Nähe zu der Magnetresonanzvorrichtung angeordnet ist, da dann ein aussagekräftiger Umfeldparameter erfasst werden kann und/oder vorliegt, der sich auch mit einer Positionsänderung der Patientenlagerungsvorrichtung bezüglich der Magnetresonanzvorrichtung ändert.

Die Vorteile des erfindungsgemäßen Verfahrens zu einer Steuerung einer Fahreinheit einer Patientenlagerungsvorrichtung mittels einer Steuerungseinheit entsprechen im Wesentlichen den Vorteilen der erfindungsgemäßen Patientenlagerungsvorrichtung, welche vorab im Detail ausgeführt sind. Hierbei erwähnte Merkmale, Vorteile oder alternative Ausführungsformen können ebenso auch auf die anderen beanspruchten Gegenstände übertragen werden und umgekehrt.

In einer vorteilhaften Weiterbildung des erfindungsgemäßen Verfahrens kann es vorgesehen sein, dass der Umfeldparameter einen Wert eines B₀-Feldes und/oder einen Wert eines Gradientenfeldes und/oder einen Wert eines B₁-Feldes und/oder eine Anzahl an Lichtmarkierungen am Ort der Patientenlagerungsvorrichtung umfasst. Vorzugsweise ist der Umfeldparameter, insbesondere der Wert des B₀-Feldes und/oder der Wert des Gradientenfeldes und/oder der Wert des B₁-Feldes und/oder die Anzahl an Lichtmarkierungen, abhängig von einer Position und/oder einem Ort der Patientenlagerungsvorrichtung bezüglich der Magnetresonanzvorrichtung, so dass derart anhand des erfassten Umfeldparameters besonders einfach eine Position der Patientenlagerungsvorrichtung bezüglich der Magnetresonanzvorrichtung ermittelt werden kann. Beispielsweise kann hierbei anhand von in der Steuerungseinheit der Patientenlagerungsvorrichtung gespeicherten und/oder hinterlegten Feldkarten des Magneten der Magnetresonanzvorrichtung eine räumliche Änderung des Magnetfelds bereitgestellt werden, die eine einfache Zuordnung des ermittelten Umfeldparameters zu einer Position der Patientenlagerungsvorrichtung innerhalb des Magnetfelds erlauben. Zudem kann eine derartige Feldkarte auch eine Verteilung und/oder Anordnung von Lichtmarkierungen an der Magnetresonanzvorrichtung und deren Sichtbarkeit in Abhängigkeit einer Position und/oder Orientierung der Patientenlagerungsvorrichtung bezüglich der Magnetresonanzvorrichtung umfassen.

Laut Erfindung wird anhand des Umfeldparameters und anhand zumindest einer Feldkarte eine Position der Patientenlagerungsvorrichtung bezüglich der Magnetresonanzvorrichtung mittels der Steuerungseinheit bestimmt. Vorzugsweise ist die zumindest eine Feldkarte in einer Speichereinheit der Patientenlagerungsvorrichtung, insbesondere in einer Speichereinheit der Steuerungseinheit, hinterlegt und/oder gespeichert.

Besonders vorteilhaft jedoch weist die Speichereinheit mehrere Feldkarten von unterschiedlichen Magneten und/oder Magnettypen von Magnetresonanzvorrichtungen auf. Vorzugsweise erfolgt eine Auswahl einer Feldkarte aus der Vielzahl an Feldkarten mittels eines Vergleichs des erfassten Umfeldparameters mit den einzelnen Feldkarten. Die Feldkarten umfassen dabei jeweils eine räumliche Änderung des Magnetfelds für einen definierten Magneten und/oder Magnettypen. Zudem kann es auch vorgesehen sein, dass zumindest eine Feldkarte über eine Kommunikationsschnittstelle der Steuerungseinheit von der Magnetresonanzvorrichtung an die Steuerungseinheit übermittelt wird. Anhand der Feldkarte und des Umfeldparameters kann somit besonders einfach und zeitsparend die Position der Patientenlagerungsvorrichtung bezüglich der Magnetresonanzvorrichtung mittels der Steuerungseinheit bestimmt werden. Beispielsweise kann dabei auch von der Steuerungseinheit bestimmt werden, auf welche Seite der Magnetresonanzvorrichtung die Patientenlagerungsvorrichtung zufährt oder ob sich die Patientenlagerungsvorrichtung vor oder hinter oder seitlich von der Magnetresonanzvorrichtung befindet. Alternativ oder zusätzlich kann auch eine Bewegungsrichtung der Patientenlagerungsvorrichtung bezüglich der Magnetresonanzvorrichtung anhand des Umfeldparameters und der zumindest einen Feldkarte bestimmt werden.

Darüber hinaus kann es vorgesehen sein, dass die Steuerung der Fahreinheit mittels der Steuerungseinheit in Abhängigkeit der zumindest eine Feldkarte erfolgt. Insbesondere kann anhand der Feldkarte und den Umfeldparametern, die bevorzugt fortlaufend bestimmt werden, sowohl ein Abstand zur Patientenlagerungsvorrichtung als auch eine Richtung der Patientenlagerungsvorrichtung bezüglich der Magnetresonanzvorrichtung ermittelt und/oder bestimmt werden. Mittels dieser Informationen kann anschließend beispielsweis eine Geschwindigkeit und/oder auch eine weitere Bewegungsrichtung der Patientenlagerungsvorrichtung mittels der Steuerungseinheit bestimmt werden und die Fahreinheit derart angesteuert werden.

Ferner kann es vorgesehen sein, dass ein Schwellwert des Umfeldparameters einstellbar ist, wobei der Schwellwert ein Kriterium für den Umfeldparameter zur Ermittlung des Steuerparameters zur Steuerung der Fahreinheit mittels der Steuerungseinheit festlegt. Vorzugsweise ist der Schwellwert manuell von einem Benutzer über eine Benutzerschnittstelle eingebbar. Mittels des Schwellwerts kann beispielsweise verhindert werden, dass die Steuerungseinheit bereits die Fahreinheit ansteuert, auch wenn die Patientenlagerungsvorrichtung noch außerhalb eines Untersuchungsraums ist und somit zwischen der Patientenlagerungsvorrichtung und der Magnetresonanzvorrichtung eine Wand angeordnet ist. Durch Eingabe und/oder Festlegung des Schwellwerts kann vorteilhaft eine hohe Sicherheit bei einem Transport der Patientenlagerungsvorrichtung erreicht werden. Der Schwellwert gibt dabei insbesondere eine Untergrenze an, und erst bei einem Erreichen und/oder Überschreiten des Schwellwerts durch den erfassten Umfeldparameter kann somit eine zumindest teilweise automatische Steuerung der Fahreinheit mittels der Steuerungseinheit erfolgen.

In einer vorteilhaften Weiterbildung der Erfindung kann es vorgesehen sein, dass die Steuerung der Fahreinheit anhand des ermittelten Steuerparameters automatisch mittels der Steuerungseinheit erfolgt, sobald ein Steuerungsparameter ermittelt wird. Hierdurch kann ein Benutzer bei einem Bewegen und/oder Fahren der Patientenlagerungsvorrichtung vorteilhaft unterstützt werden. Insbesondere kann hierbei der Benutzer bei einem Andockvorgang, bei dem eine exakte Position der Patientenlagerungsvorrichtung bezüglich der Magnetresonanzvorrichtung erforderlich ist, vorteilhaft unterstützt werden. Während des Andockvorgangs befindet sich vorteilhafterweise die Patientenlagerungsvorrichtung im Magnetfeld des Magneten, so dass eine Steuerung des Andockvorgangs in Abhängigkeit des Umfeldparameters erfolgen kann. Vorzugsweise wird dem Benutzer dabei auch angezeigt, dass die Fahreinheit von der Steuerungseinheit automatisch gesteuert wird, so dass der Benutzer sich in dieser Zeit, insbesondere während des Andockvorgangs, weiteren Tätigkeiten zuwenden kann. Mittels der automatischen Steuerung der Fahreinheit durch die Steuerungseinheit kann zudem eine vorteilhafte Zeitersparnis für den Benutzer bei einer Vorbereitung einer Magnetresonanzuntersuchung erreicht werden.

Alternativ oder zusätzlich kann die Steuerung der Fahreinheit anhand des ermittelten Steuerparameters automatisch von der Steuerungseinheit in Abhängigkeit einer Benutzeraktion erfolgen. Dies ermöglicht eine vorteilhafte Unterstützung des Benutzers bei einem Bewegen der Patientenlagerungsvorrichtung, wobei der Benutzer dabei eine Kontrolle der Steuerung mittels der Benutzeraktion beibehält. Beispielsweise kann dies durch Drücken einer "Vorwärts"-Taste oder durch Drücken einer "Dock-On"-Taste und/oder weiterer, dem Fachmann als sinnvoll erscheinender Tasten durch den Benutzer erfolgen.

Des Weiteren kann es vorgesehen sein, dass anhand des Umfeldparameters eine Höhe des Liegentischs mittels der Steuerungseinheit bestimmt wird. Dies weist den Vorteil auf, dass eine Position und/oder ein Ort des Liegentischs im Raum bestimmt werden kann. Zudem kann der Liegentisch der Patientenlagerungsvorrichtung bereits vor einem Andocken an die Magnetresonanzvorrichtung auf eine für den Andockvorgang erforderliche Höhe eingestellt werden. Ein Unterbrechen des Andockvorgangs, um den Liegentisch der Patientenlagerungsvorrichtung auf die erforderliche Höhe zu bewegen, kann somit entfallen. Vorzugsweise wird mittels der ermittelten und/oder bestimmten Höhe des Liegentischs der Patientenlagerungsvorrichtung eine für den Andockvorgang erforderliche Höhe von der Steuerungseinheit automatisch eingestellt.

Vorteilhafterweise kann anhand des Umfeldparameters eine Bauart eines Magneten der Magnetresonanzvorrichtung mittels der Steuerungseinheit bestimmt wird. Anhand der Bauart des Magneten kann vorteilhaft eine räumliche Änderung des Magnetfelds insbesondere außerhalb der Magnetresonanzvorrichtung ermittelt werden. Die räumliche Änderung des Magnetfelds insbesondere außerhalb der Magnetresonanzvorrichtung kann dabei abhängig sein von der Bauart des Magneten. Anhand der räumlichen Änderung des Magnetfelds zusammen mit dem erfassten Umfeldparameter kann eine besonders einfache Bestimmung und/oder Ermittlung der Position und/oder der Bewegungsrichtung der Patientenlagerungsvorrichtung erreicht werden. Vorzugsweise wird zur Bestimmung der Bauart des Magneten der Umfeldparameter mit in der Steuerungseinheit hinterlegten Feldkarten verglichen.

Des Weiteren geht die Erfindung aus von einem Computerprogrammprodukt, welches ein Programm umfasst und direkt in einem Speicher einer programmierbaren Steuerungseinheit einer Patientenlagerungsvorrichtung ladbar ist, mit Programmmitteln, um ein Verfahren zu einer Steuerung einer Fahreinheit einer Patientenlagerungsvorrichtung auszuführen, wenn das Programm in der Steuerungseinheit der Patientenlagerungsvorrichtung ausgeführt wird. Dabei benötigt das Computerprogramm eventuell Programmmittel, z.B. Bibliotheken und Hilfsfunktionen, um die entsprechenden Ausführungsformen des Verfahrens zu realisieren. Das Computerprogramm kann dabei eine Software mit einen Quellcode, der noch compiliert und gebunden oder der nur interpretiert werden muss, oder einen ausführbaren Softwarecode umfassen, der zur Ausführung nur noch in eine entsprechende Recheneinheit zu laden ist.

Des Weiteren geht die Erfindung aus von einem computerlesbaren Datenträger, welcher ein Programm umfasst, das zu einer Ausführung eines Verfahrens zu einer Steuerung einer Fahreinheit einer Patientenlagerungsvorrichtung vorgesehen ist.

Weitere Vorteile, Merkmale und Einzelheiten der Erfindung ergeben sich aus dem im Folgenden beschriebenen Ausführungsbeispiel sowie anhand der Zeichnungen.

Es zeigen:
- Fig. 1: eine erfindungsgemäße Patientenlagerungsvorrichtung und eine Magnetresonanzvorrichtung in einer schematischen Seitenansicht,
- Fig. 2: die Patientenlagerungsvorrichtung und die Magnetresonanzvorrichtung in einer schematischen Darstellung,
- Fig. 3: die Patientenlagerungsvorrichtung und die Magnetresonanzvorrichtung in einer schematischen Draufsicht und
- Fig. 4: ein Ablaufdiagramm eines erfindungsgemäßen Verfahrens zu einer Steuerung einer Fahreinheit einer Patientenlagerungsvorrichtung.

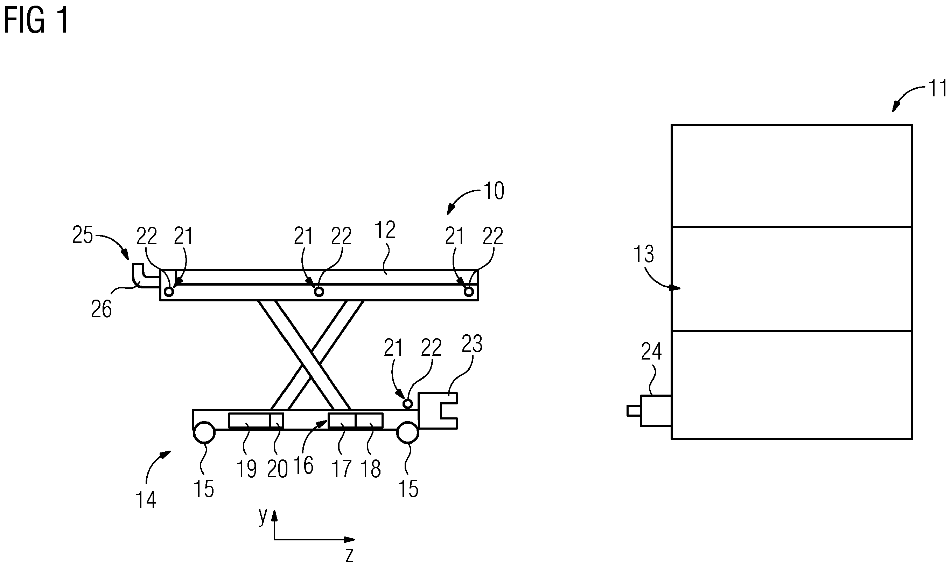

In den Fig. 1 bis 3 ist schematisch eine erfindungsgemäße Patientenlagerungsvorrichtung 10 und eine Magnetresonanzvorrichtung 11 dargestellt. Die Patientenlagerungsvorrichtung 10 umfasst einen Liegentisch 12, der, sobald die Patientenlagerungsvorrichtung 10 an die Magnetresonanzvorrichtung 11 angedockt ist, innerhalb eines Patientenaufnahmebereichs 13 der Magnetresonanzvorrichtung 11 bewegbar ist.

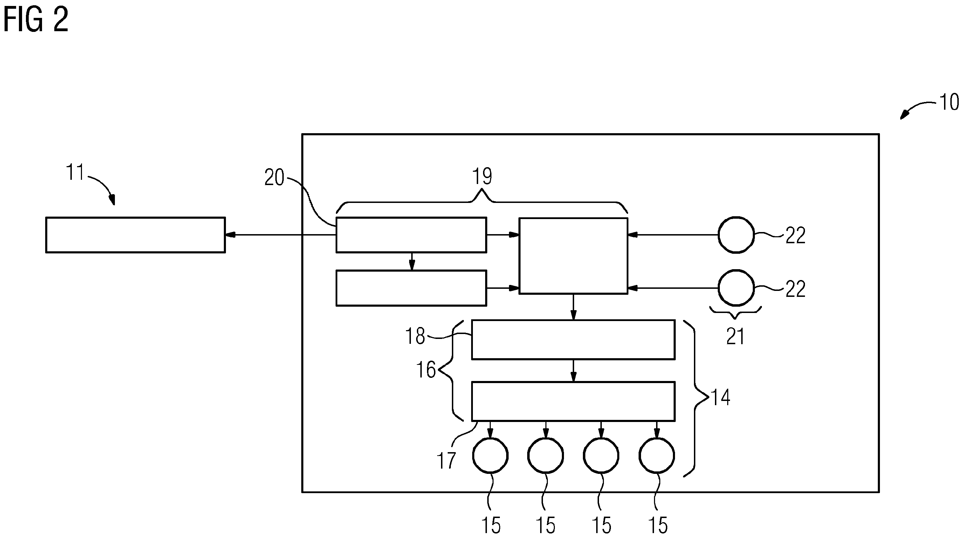

Die Patientenlagerungsvorrichtung 10 umfasst zudem eine Fahreinheit 14 (Fig. 1 und 2), die an einem dem Liegentisch 12 abgewandten Bereich an der Patientenlagerungsvorrichtung 10 angeordnet ist. Die Fahreinheit 14 ist zu einer Bewegung der Patientenlagerungsvorrichtung 10 ausgelegt und weist mehrere Rollelemente 15 und/oder Rollkörper auf, wobei in Fig. 1 nur zwei der mehreren Rollelemente 15 und/oder Rollkörper zu sehen sind. Bevorzugt weist die Fahreinheit vier oder fünf Rollelemente 15 und/oder Rollkörper auf. Die einzelnen Rollelemente 15 und/oder Rollkörper sind jeweils von Rädern gebildet.

Des Weiteren umfasst die Fahreinheit 14 auch eine Antriebseinheit 16 mit einer Motoreinheit 17 und einer Motortreibereinheit 18 (Fig. 2). Mittels der Motortreibereinheit 18 erfolgt eine Ansteuerung der Motoreinheit 17. Die Motoreinheit 17 erzeugt ein Antriebsmoment, mittels dessen die einzelnen Rollelemente 15 und/oder Rollkörper der Fahreinheit 14 angesteuert werden. Bevorzugterweise sind die einzelnen Rollelemente 15 und/oder Rollkörper einzelnen von der Motoreinheit 17 ansteuerbar.

Des Weiteren umfasst die Patientenlagerungsvorrichtung 10 eine Steuerungseinheit 19 (Fig. 1 und 2). Mittels der Steuerungseinheit 19 erfolgt eine Steuerung der Fahreinheit 14, insbesondere der Motortreibereinheit 18 und der Motoreinheit 17. Die Steuerungseinheit 19 umfasst eine zentrale Steuerungseinheit 19 der Patientenlagerungsvorrichtung 10, mittels der auch eine Höheneinstelleinheit für den Liegentisch 12 angesteuert werden kann. Die Steuerungseinheit 19 weist eine Kommunikationsschnittstelle 20 auf für eine Kommunikation mit der Magnetresonanzvorrichtung 11. Die Kommunikationsschnittstelle 20 ist bevorzugt kabellos und/oder drahtlos ausgebildet. Im vorliegenden Ausführungsbeispiel umfasst die Kommunikationseinheit 20 eine Funkverbindung zu der Magnetresonanzvorrichtung 11. Grundsätzlich sind weitere Ausgestaltungen der Kommunikationseinheit zur Kommunikation zwischen der Steuerungseinheit 19 der Patientenlagerungsvorrichtung 10 und der Magnetresonanzvorrichtung 11 in einer alternativen Ausgestaltung der Erfindung denkbar.

Zu einer Erfassung zumindest eines Umfeldparameters UP der Patientenlagerungsvorrichtung 10 weist die Patientenlagerungsvorrichtung 10 eine Sensoreinheit 21 auf (Fig. 1 bis 3). Die Sensoreinheit 21 umfasst mehrere Umfeldsensoren, die im vorliegenden Ausführungsbeispiel von jeweils einem B₀-Feldsensor 22 gebildet sind. Die einzelnen B₀-Feldsensoren 22 sind bevorzugt von jeweils einem Hall-Sensor gebildet. Die einzelnen B₀-Feldsensoren 22 sind jeweils zu einer Erfassung eines Umfeldparameters UP der Patientenlagerungsvorrichtung 10 ausgelegt. Insbesondere sind die einzelnen B₀-Feldsensoren 22 zu einer Erfassung eines Werts eines B₀-Feldes und/oder eines Magnetfeldstärke am Ort der Patientenlagerungsvorrichtung ausgelegt. Zudem sind die einzelnen B₀-Feldsensoren 22 verteilt an der Patientenlagerungsvorrichtung 10 angeordnet. Bevorzugt sind die einzelnen B₀-Feldsensoren 22 in einem Frontbereich der Patientenlagerungsvorrichtung 10 oder an dem Liegentisch 12 der Patientenlagerungsvorrichtung 10 angeordnet. Die mehreren B₀-Feldsensoren 22 sind zudem beabstandet zueinander angeordnet, so dass eine hohe Genauigkeit und/oder eine Eindeutigkeit der erfassten Umfeldparameter UP erreicht werden kann.

In einer alternativen Ausgestaltung der Erfindung können die einzelnen Umfeldsensoren auch einen Magnetfeldsensor aufweisen, der zur Erfassung eines Gradientenfelds und/oder zur Erfassung eines B₁-Feldes ausgelegt ist. Auch eine Ausgestaltung der einzelnen Umfeldsensoren zur Erfassung von Lichtmarkierungen kann in einer weiteren Ausgestaltung der Erfindung vorgesehen sein. Im vorliegenden jedoch wird die Erfindung beispielhaft anhand der Ausbildung der einzelnen Umfeldsensoren als B₀-Feldsensoren 22, insbesondere Hall-Sensoren, beschrieben. Eine Ausgestaltung der Patientenlagerungsvorrichtung bei einer Ausbildung der Umfeldsensoren als Magnetfeldsensoren zur Erfassung eines Gradientenfelds und/oder eines B₁-Feldes und/oder bei einer Ausbildung der Umfeldsensoren als Sensoren zur Erfassung von Lichtmarkierungen erfolgt analog zur Ausgestaltung der Umfeldsensoren als B₀-Feldsensoren 22, so dass hierzu auf die vorliegende Beschreibung des vorliegenden Ausführungsbeispiels verwiesen wird.

In dem Frontbereich der Patientenlagerungsvorrichtung 10 ist eine Andockeinheit 23 angeordnet, die korrespondierend zu einer Andockeinheit 24 der Magnetresonanzvorrichtung 11 ausgebildet ist. Die Andockeinheit 23 der Patientenlagerungsvorrichtung 10 ist dazu ausgelegt, zusammen mit der Andockeinheit 24 der Magnetresonanzvorrichtung 11 ein Andocken der Patientenlagerungsvorrichtung 10 an die Magnetresonanzvorrichtung 11 zu ermöglichen. Zumindest ein B₀-Feldsensor 22 oder auch mehrere B₀-Feldsensoren 22 sind in diesem Frontbereich in der Nähe zur Andockeinheit 23 der Patientenlagerungsvorrichtung 10 angeordnet, wobei in Fig. 1 nur ein einziger B₀-Feldsensor 22 zu sehen ist. Der Liegentisch 12 der Patientenlagerungsvorrichtung 10 umfasst ebenfalls mehrere B₀-Feldsensoren 22, die verteilt an dem Liegentisch 12 angeordnet sind. Insbesondere sind die B₀-Feldsensoren 22 in einem Frontbereich des Liegentischs 12 und/oder in einem Randbereich des Liegentischs 12 angeordnet. Beispielsweise sind die B₀-Feldsensoren 22 um eine Liegefläche des Liegentischs 12 verteilt angeordnet.

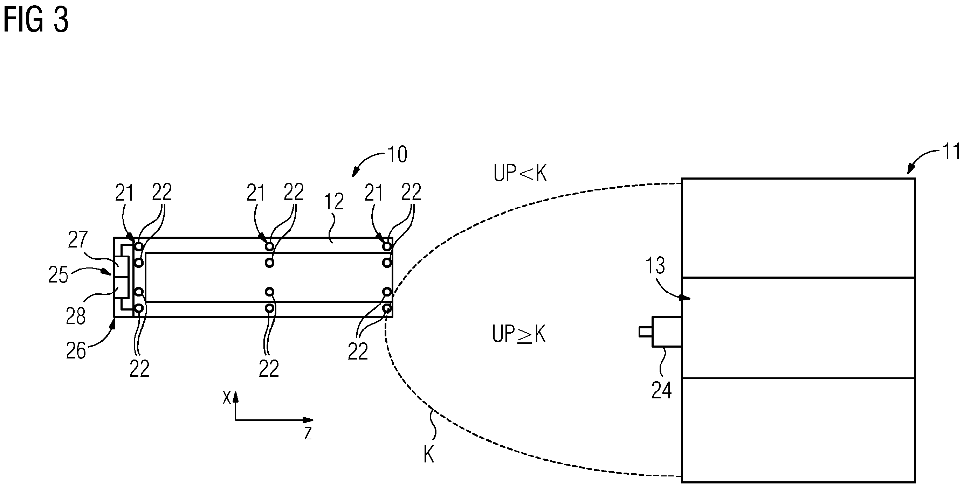

Die Patientenlagerungsvorrichtung 10 weist weiterhin eine Benutzerschnittstelle 25 auf, die in einem Griffbereich 26 der Patientenlagerungsvorrichtung 10 angeordnet ist (Fig. 1 und 3). Die Benutzerschnittstelle 25 weist bevorzugt eine Eingabeeinheit 27 auf, mittels der der Benutzer Eingaben für eine Bedienung der Patientenlagerungsvorrichtung 10 tätigen kann. Zudem weist die Benutzerschnittstelle 25 auch eine 28 auf zu einer Übermittlung einer Information der Patientenlagerungsvorrichtung 10 an den Benutzer.

Die erfassten Umfeldparameter UP umfassen eine Magnetfeldstärke und/oder einen Wert eines B₀-Feldes, die das von dem Magneten der Magnetresonanzvorrichtung 11 erzeugte Magnetfeld am Ort der Patientenlagerungsvorrichtung 10 aufweist. Von der Steuerungseinheit 19 wird anhand der erfassten Umfeldparameter UP zumindest ein Steuerparameter ermittelt, wobei mittels des ermittelten Steuerparameters eine Steuerung der Fahreinheit 14 erfolgen kann. Hierbei ist die Steuerungseinheit 19 dazu ausgelegt, dass zunächst eine Position der Patientenlagerungsvorrichtung bezüglich der Magnetresonanzvorrichtung 11 anhand der erfassten Umfeldparameter UP ermittelt wird. Des Weiteren ist die Steuerungseinheit 19 dazu ausgelegt, ausgehend von der Position der Patientenlagerungsvorrichtung 10 einen Steuerungsparameter zu generieren und/oder zu ermitteln, mittels dessen die Fahreinheit 14, insbesondere die Antriebseinheit 16 der Fahreinheit 14, angesteuert wird.

Mittels der Benutzerschnittstelle 25 kann zudem von einem Benutzer ein Schwellwert K für die Umfeldparameter UP eingestellt und/oder eingegeben werden. Der Schwellwert K legt dabei ein Kriterium für den Umfeldparameter UP zur Ermittlung des Steuerparameters zur Steuerung der Fahreinheit 14 mittels der Steuerungseinheit 19 fest. Das Kriterium des Umfeldparameters UP gibt insbesondere eine Untergrenze für den Umfeldparameter UP an, ab der eine Ermittlung des zumindest einen Steuerparameters in Abhängigkeit von dem Umfeldparameter UP erfolgt. Beispielsweise umfasst das Kriterium eine Untergrenze für einen Wert der Magnetfeldstärke (Fig. 3). Liegt der Umfeldparameter UP unterhalb des gewählten und/oder eingestellten Kriteriums, also unterhalb der Untergrenze für die erfassten Umfeldparameter UP, erfolgt von der Steuerungseinheit 19 somit keine Ermittlung eines Steuerparameters in Abhängigkeit von dem Umfeldparameter UP. Ist dagegen der erfasste Umfeldparameter UP größer oder gleich dem gewählten und/oder eingestellten Kriteriums, also größer oder gleich der Untergrenze für die erfassten Umfeldparameter UP, erfolgt von der Steuerungseinheit 19 somit eine Ermittlung eines Steuerparameters in Abhängigkeit von dem Umfeldparameter UP.

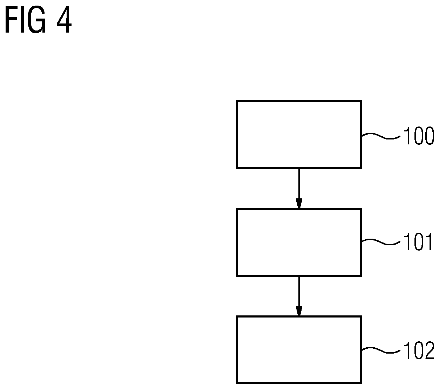

In Fig. 4 ist ein Verfahren zur Steuerung der Fahreinheit 14 der Patientenlagerungsvorrichtung 10 mittels der Steuerungseinheit 19 dargestellt. Zu einer Steuerung der Fahreinheit 14 weist die Steuerungseinheit 19 Computerprogramme und/oder eine Software auf, die direkt in einer Speichereinheit ladbar sind, mit Programmmitteln, um das Verfahren zur Steuerung der Fahreinheit 14 der Patientenlagerungsvorrichtung 10 auszuführen, wenn die Computerprogramme und/oder Software in der Steuerungseinheit 19 ausgeführt werden. Die Steuerungseinheit 19 weist hierzu einen nicht näher dargestellten Prozessor, der zu einer Ausführung der Computerprogramme und/oder Software ausgelegt ist, und die Speichereinheit auf, in der die Software und/oder Computerprogramme hinterlegt und/oder gespeichert sind.

Alternativ oder zusätzlich können die Software und/oder Computerprogramme auch auf einem separat zur Steuerungseinheit 19 ausgebildeten elektronisch lesbaren Datenträger und/oder einer Cloud gespeichert und/oder hinterlegt sein. Hierbei kann die Steuerungseinheit 19 mittels eines Datennetzes auf den elektronisch lesbaren Datenträger zugreifen.

Das Verfahren zur Steuerung der Fahreinheit 14 der Patientenlagerungsvorrichtung 10 mittels der Steuerungseinheit 19 wird beispielhaft anhand der Ausbildung der einzelnen Umfeldsensoren als B₀-Feldsensoren 22, insbesondere Hall-Sensoren, beschrieben. Das Verfahren zur Steuerung der Fahreinheit 14 der Patientenlagerungsvorrichtung 10 mittels der Steuerungseinheit 19 wird bei einer alternativen Ausbildung der Umfeldsensoren als Magnetfeldsensoren zur Erfassung eines Gradientenfelds und/oder eines B₁-Feldes und/oder bei einer Ausbildung der Umfeldsensoren als Sensoren zur Erfassung von Lichtmarkierungen analog ausgeführt, so dass hier auf die folgende Beschreibung des vorliegenden Ausführungsbeispiels verwiesen wird.

In einem ersten Verfahrensschritt 100 des Verfahrens zur Steuerung der Fahreinheit 14 mittels der Steuerungseinheit 19 wird zunächst zumindest ein Umfeldparameter UP der Patientenlagerungsvorrichtung 10 mittels der Sensoreinheit 21 erfasst. Hierbei erfassen die einzelnen B₀-Feldsensoren 22 der Sensoreinheit 21 jeweils einen Umfeldparameter UP, insbesondere einen Wert eines B₀-Feldes, am Ort der Patientenlagerungsvorrichtung 10. Der von den einzelnen B₀-Feldsensoren 22 erfassten Umfeldparameter UP sind zudem Abhängig von einer Position der Patientenlagerungsvorrichtung 10 bezüglich der Magnetresonanzvorrichtung 11. Befindet sich die Patientenlagerungsvorrichtung 10 mit geringem Abstand von beispielsweise 1 m bis 2 m zur Magnetresonanzvorrichtung 11, insbesondere in einem Frontbereich oder einem Heckbereich der Magnetresonanzvorrichtung 11, so sind auch die einzelnen B₀-Feldsensoren 22 mit geringem Abstand zur Magnetresonanzvorrichtung 11 angeordnet und erfassen somit einen Umfeldparameter UP mit einen großen Wert der Magnetfeldstärke und/oder ein großes B₀-Feld. Ist die Patientenlagerungsvorrichtung 10 dagegen mit einem großen Abstand zur Magnetresonanzvorrichtung 11 angeordnet, so sind somit auch die einzelnen B₀-Feldsensoren 22 mit einem großen Abstand zur Magnetresonanzvorrichtung 11 angeordnet und erfassen somit einen Umfeldparameter UP mit einem kleinen Wert der Magnetfeldstärke und/oder ein kleines B₀-Feld.

In einem daran anschließenden Verfahrensschritt 101 wird von der Steuerungseinheit 19 in Abhängigkeit des zumindest einen erfassten Umfeldparameters UP, insbesondere in Abhängigkeit der mehreren erfassten Umfeldparameter UP, ein Steuerparameter für eine Ansteuerung der Fahreinheit 14 ermittelt. Für eine Ermittlung des Steuerparameters kann zudem von einem Benutzer in diesem Verfahrensschritt 101 ein Schwellwert K mittels der Eingabeeinheit 27 der Benutzerschnittstelle 25 eingegeben werden. Der Schwellwert K legt dabei ein Kriterium für den Umfeldparameter UP zur Ermittlung des Steuerparameters zur Steuerung der Fahreinheit 14 fest. Mittels des Schwellwerts K kann somit ein Wert der Magnetfeldstärke vom Benutzer festgelegt werden, ab dem eine Ermittlung eines Steuerparameters zur Steuerung der Fahreinheit 14 mittels der Steuerungseinheit 19 erfolgt. Somit erfolgt die Ermittlung des Steuerparameters zur Steuerung der Fahreinheit 14 erst, sobald ein erfasster Umfeldparameter UP gleich dem Schwellwert K oder auch größer als der Schwellwert K ist. Ist hingegen ein erfasster Umfeldparameter UP kleiner als der Schwellwert K, erfolgt für diesen Umfeldparameter UP keine Ermittlung eines Steuerparameters zur Steuerung der Fahreinheit 14. Somit kann vom Benutzer festgelegt werden, ab wann er eine Unterstützung bei einem Bewegen der Patientenlagerungsvorrichtung 10 haben möchte.

Zudem kann der Schwellwert K bereits mit einer Untergrenze vorbelegt sein, so dass von einem Benutzer nur noch ein nach oben veränderbarer Schwellwert K eingegeben werden kann. Eine derartige Vorbelegung des Schwellwerts K kann sinnvoll sein, um eine automatische und/oder selbsttätige Steuerung der Fahreinheit 14 mittels der Steuerungseinheit 19 noch außerhalb des Untersuchungsraums, in dem die Magnetresonanzvorrichtung 11 angeordnet ist, zu verhindern.

In diesem Verfahrensschritt 101 des Ermittelns zumindest eines Steuerparameters wird zunächst, sofern die erfassten Umfeldparameter UP größer als der Schwellwert K sind, eine Position der Patientenlagerungsvorrichtung 10 bezüglich der Magnetresonanzvorrichtung 11 anhand der erfassten Umfeldparameter UP ermittelt. Hierbei werden die von den einzelnen B₀-Feldsensoren 22 erfassten Umfeldparameter UP mit in der Steuerungseinheit 19 hinterlegten und/oder gespeicherten Feldkarten, insbesondere Magnetfeldkarten, verglichen. Innerhalb der Steuerungseinheit 19, insbesondere innerhalb der Speichereinheit der Steuerungseinheit 19, sind mehrere Felskarten von unterschiedlichen Magnettypen von unterschiedlichen Magnetresonanzvorrichtungen 11 gespeichert. Durch einen Vergleich der erfassten Umfeldparameter UP mit den einzelnen Feldkarten und diejenige Feldkarte mit der größten Übereinstimmung mit den vorzugsweise mehreren Umfeldparametern UP ausgewählt. Insbesondere kann hierbei aufgrund der innerhalb der Patientenlagerungsvorrichtung 10 verteilt angeordneten B₀-Feldsensoren 22 eine räumliche Verteilung der erfassten Umfeldparameter UP mit den Feldkarten verglichen werden und daraus diejenige Feldkarte mit den größten Übereinstimmungen mit den mehreren Umfeldparametern UP ausgewählt.

Anhand der ausgewählten Feldkarte und den erfassten Umfeldparametern UP kann somit eine Position der Patientenlagerungsvorrichtung 10 bezüglich der Magnetresonanzvorrichtung 11 mittels der Steuerungseinheit 19 bestimmt werden. Zudem kann auch eine Bewegungsrichtung und/oder Orientierung der Patientenlagerungsvorrichtung 10 bezüglich der Magnetresonanzvorrichtung 10 anhand der ausgewählten Feldkarte und den erfassten Umfeldparametern UP bestimmt werden. Auch kann durch Auswählen einer Feldkarte ebenfalls eine Magnettyp und/oder eine Bauart des Magneten der Magnetresonanzvorrichtung 11 bestimmt werden.

Des Weiteren kann in diesem Verfahrensschritt 101 des Ermittelns zumindest eines Steuerparameters auch eine Auswahl einer Feldkarte über eine Kommunikation der Steuerungseinheit 19 der Patientenlagerungsvorrichtung 10 mit der Magnetresonanzvorrichtung 11 erfolgen. Hierzu kann beispielsweise mittels der Kommunikationseinheit 20 der Steuerungseinheit 19 ein Informationsaustausch mit der Magnetresonanzvorrichtung 11 erfolgen und dabei eine Information einer Feldkarte und/oder einer Feldverteilung des Magneten der Magnetresonanzvorrichtung 11 an die Steuerungseinheit 19 übermittelt werden.

Zudem kann es in einer weiteren Ausgestaltung der Erfindung auch vorgesehen sein, dass die von der Steuerungseinheit 19 ausgewählte Feldkarte auch zu einer Festlegung des Schwellwerts K für die Umfeldparameter UP herangezogen wird. Insbesondere eine Vorbelegung und/oder eine Voreinstellung des Schwellwerts K kann mittels der Steuerungseinheit 19 anhand der ausgewählten Feldkarte erfolgen.

Anhand des ermittelten Abstands und/oder der ermittelten Orientierung der Patientenlagerungsvorrichtung 10 bezüglich der Magnetresonanzvorrichtung 11 und der ausgewählten Feldkarte wird von der Steuerungseinheit 19 zumindest ein Steuerparameter zur Ansteuerung der Fahreinheit 14 generiert und/oder ermittelt. Der Steuerparameter kann dabei eine Steuerinformation zur Ansteuerung der Antriebseinheit 16 umfassen. Insbesondere kann dabei der Steuerparameter zur Ansteuerung der Antriebseinheit 16 eine Steuerinformation einer einzustellenden Geschwindigkeit und/oder eine Steuerinformation einer einzustellenden Bewegungsrichtung der Rollelemente 15 und/oder Rollkörper umfassen.

In dem Verfahrensschritt 101 des Ermittelns zumindest eines Steuerparameters kann zudem auch eine Höhe des Liegentischs 12 mittels der Steuerungseinheit 19 bestimmt werden. Diese Information kann dabei insbesondere anhand der erfassten Umfeldparameter UP der innerhalb des Liegentischs 12 angeordneten B₀-Feldsensoren 22 erfolgen.

In einem weiteren Verfahrensschritt 102 erfolgt ein Ansteuern der Fahreinheit 14 mittels der Steuerungseinheit 19 anhand des zumindest einen ermittelten und/oder generierten Steuerparameters. Die Steuerung der Fahreinheit 14, insbesondere der Antriebseinheit 16, kann dabei vollständig automisch mittels der Steuerungseinheit 19 anhand des zumindest einen Steuerparameters erfolgen, sobald ein Steuerparameter mittels der Steuerungseinheit 19 ermittelt und/oder generiert wird. Dies ermöglicht eine im Wesentlichen automatische Navigation der Patientenlagerungsvorrichtung 10 in der Nähe von der Magnetresonanzvorrichtung 11, ohne dass hierzu eine Benutzeraktion erforderlich ist. Insbesondere kann derart eine automatische und/oder selbsttätige Navigation für einen Andockvorgang der Patientenlagerungsvorrichtung 10 an die Magnetresonanzvorrichtung 11 bereitgestellt werden. Hierbei sind die Steuerparameter zur Ansteuerung der Fahreinheit 14 derart ausgebildet, dass eine exakte Positionierung der Patientenlagerungsvorrichtung 10 für den Andockvorgang an der Magnetresonanzvorrichtung 11 erreicht wird.

Sobald eine automatische Navigation der Patientenlagerungsvorrichtung 10 gesteuert durch die Steuerungseinheit 19 anhand der ermittelten und/oder generierten Steuerparameter erfolgt, wird zudem eine Ausgabeinformation von der Steuerungseinheit 19 generiert. Diese Ausgabeinformation wird über die Benutzerschnittstelle 25, insbesondere der Ausgabeeinheit 28 der Benutzerschnittstelle 25, an den Benutzer mitgeteilt, so dass der Benutzer die Zeit des automatischen Navigierens und/oder des automatischen Andockens der Patientenlagerungsvorrichtung 10 gesteuert mittels der Steuerungseinheit 19 für weitere Tätigkeiten nutzen kann.

Alternativ oder zusätzlich ist es auch möglich, dass in diesem weiteren Verfahrensschritt 102 des Ansteuerns der Fahreinheit 14 mittels der Steuerungseinheit 19 anhand des zumindest einen ermittelten und/oder generierten Steuerparameters eine Steuerung der Fahreinheit 14, insbesondere der Antriebseinheit 16, nur teilweise automatisch mittels der Steuerungseinheit 19 anhand des zumindest einen Steuerparameters erfolgt, sobald ein Steuerparameter mittels der Steuerungseinheit 19 ermittelt und/oder generiert wird. Hierzu ist eine Benutzeraktion durch den Benutzer mittels der Benutzerschnittstelle 25, insbesondere der Eingabeeinheit 27 der Benutzerschnittstelle 25, erforderlich. Beispielsweise hierbei der Benutzer lediglich eine Taste, wie insbesondere eine "Vorwärts"-Taste, eine "Andock"-Taste usw. drücken und/oder gedrückt halten. Der weitere Navigationsvorgang und/oder Andockvorgang dagegen wird automatisch und/oder selbsttätig von der Steuerungseinheit 19 anhand der ermittelten und/oder generierten Steuerparameter ausgeführt.

Obwohl die Erfindung im Detail durch das bevorzugte Ausführungsbeispiel näher illustriert und beschrieben wurde, so ist die Erfindung nicht durch die offenbarten Beispiele eingeschränkt und andere Variationen können vom Fachmann hieraus abgeleitet werden, ohne den Schutzumfang der Erfindung zu verlassen.

## Patentansprüche

1. Patientenlagerungsvorrichtung (10) mit einem Liegentisch (12), einer Fahreinheit (14) die zu einer Bewegung der Patientenlagerungsvorrichtung ausgelegt ist, einer Steuerungseinheit (19) und einer Sensoreinheit (21) wobei die Sensoreinheit zumindest einen Umfeldparameter der Patientenlagerungsvorrichtung erfasst und die Steuerungseinheit in Abhängigkeit des zumindest einen Umfeldparameters einen Steuerparameter zur Steuerung der Fahreinheit ermittelt, wobei die Sensoreinheit zumindest einen Umfeldsensor aufweist, der einen B0-Feldsensor umfasst, und wobei die Patientenlagerungsvorrichtung einen Frontbereich mit einer Andockvorrichtung zu einem Andocken an einer Magnetresonanzvorrichtung aufweist, wobei die Sensoreinheit in dem Frontbereich angeordnet ist.

2. Patientenlagerungsvorrichtung nach Anspruch 1,
**dadurch gekennzeichnet, dass** der zumindest eine B₀-Feldsensor einen Hallsensor umfasst.

3. Patientenlagerungsvorrichtung nach einem der vorhergehenden Ansprüche,
**dadurch gekennzeichnet, dass** die Sensoreinheit zumindest zwei Umfeldsensoren aufweist, die beabstandet zueinander angeordnet sind.

4. Patientenlagerungsvorrichtung nach einem der vorhergehenden Ansprüche,
**dadurch gekennzeichnet, dass** die Sensoreinheit zumindest einen Umfeldsensor aufweist, der innerhalb des Liegentischs angeordnet ist.

5. Patientenlagerungsvorrichtung nach einem der vorhergehenden Ansprüche,
**gekennzeichnet durch** eine Benutzerschnittstelle, die zu einem Einstellen eines Schwellwerts des Umfeldparameters ausgelegt ist, wobei der Schwellwert ein Kriterium für den Umfeldparameter zur Ermittlung des Steuerparameters zur Steuerung der Fahreinheit mittels der Steuerungseinheit festlegt.

6. Patientenlagerungsvorrichtung nach einem der vorhergehenden Ansprüche,
**dadurch gekennzeichnet, dass** die Steuerungseinheit eine Kommunikationsschnittstelle für eine Kommunikation mit einer Magnetresonanzvorrichtung aufweist.

7. Magnetresonanzvorrichtung mit einer Patientenlagerungsvorrichtung nach einem der Ansprüche 1 bis 6.

8. Verfahren zu einer Steuerung einer Fahreinheit einer Patientenlagerungsvorrichtung mittels einer Steuerungseinheit während eines Andockvorgangs der Patientenlagerungsvorrichtung an eine Magnetresonanzvorrichtung, umfassend die folgenden Schritte:
- Erfassen von zumindest einem Umfeldparameter, wobei der Umfeldparameter einen Wert eines B₀-Feldes umfasst, der Patientenlagerungsvorrichtung mittels einer Sensoreinheit, wobei die Sensoreinheit zumindest einen Umfeldsensor aufweist, der einen B₀-Feldsensor umfasst,
- Ermitteln von zumindest einen Steuerparameter für eine Ansteuerung der Fahreinheit mittels der Steuerungseinheit, wobei die Ermittlung des zumindest einen Steuerparameters in Abhängigkeit des zumindest einen Umfeldparameters erfolgt, wobei anhand des Umfeldparameters und anhand zumindest einer Feldkarte eine Position der Patientenlagerungsvorrichtung bezüglich der Magnetresonanzvorrichtung mittels der Steuerungseinheit bestimmt wird, und
- Ansteuerung der Fahreinheit gemäß dem ermittelten Steuerparameter mittels der Steuerungseinheit.

9. Verfahren nach Anspruch 8,
**dadurch gekennzeichnet, dass** der Umfeldparameter einen Wert eines Gradientenfeldes und/oder einen Wert eines B₁-Feldes und/oder eine Anzahl an Lichtmarkierungen am Ort der Patientenlagerungsvorrichtung umfasst.

10. Verfahren nach einem der Ansprüche 8bis 9,
**dadurch gekennzeichnet, dass** die Steuerung der Fahreinheit mittels der Steuerungseinheit in Abhängigkeit der zumindest einen Feldkarte erfolgt.

11. Verfahren nach einem der Ansprüche 8 bis 10,
**dadurch gekennzeichnet, dass** ein Schwellwert des Umfeldparameters einstellbar ist, wobei der Schwellwert ein Kriterium für den Umfeldparameter zur Ermittlung des Steuerparameters zur Steuerung der Fahreinheit mittels der Steuerungseinheit festlegt.

12. Verfahren nach einem der Ansprüche 8 bis 11,
**dadurch gekennzeichnet, dass** die Steuerung der Fahreinheit anhand des ermittelten Steuerparameters automatisch von der Steuerungseinheit erfolgt, sobald ein Steuerparameter ermittelt wird.

13. Verfahren nach einem der Ansprüche 8 bis 12,
**dadurch gekennzeichnet, dass** die Steuerung der Fahreinheit anhand des ermittelten Steuerparameters automatisch von der Steuerungseinheit in Abhängigkeit einer Benutzeraktion erfolgt.

14. Verfahren nach einem der Ansprüche 8 bis 13,
**dadurch gekennzeichnet, dass** anhand des Umfeldparameters eine Höhe des Liegentischs mittels der Steuerungseinheit bestimmt wird.

15. Verfahren nach einem der Ansprüche 8 bis 14,
**dadurch gekennzeichnet, dass** anhand des Umfeldparameters eine Bauart eines Magneten der Magnetresonanzvorrichtung mittels der Steuerungseinheit bestimmt wird.

16. Verfahren nach Anspruch 15,
**dadurch gekennzeichnet, dass** zur Bestimmung der Bauart des Magneten der Umfeldparameter mit in der Steuerungseinheit hinterlegten Feldkarten verglichen wird.

17. Verfahren nach einem der Ansprüche 8 bis 16,
**dadurch gekennzeichnet, dass** anhand des Umfeldparameters eine Bewegungsrichtung der Patientenlagerungsvorrichtung mittels der Steuerungseinheit bestimmt wird.

18. Computerprogrammprodukt, welches ein Programm umfasst und direkt in einen Speicher einer programmierbaren Steuerungseinheit einer Patientenlagerungsvorrichtung ladbar ist, mit Programmmitteln, um ein Verfahren zu einer Steuerung einer Fahreinheit einer Patientenlagerungsvorrichtung nach einem der Ansprüche 8 bis 17 auszuführen, wenn das Programm in der Steuerungseinheit der Patientenlagerungsvorrichtung ausgeführt wird.

19. Elektronisch lesbarer Datenträger mit darauf gespeicherten elektronisch lesbaren Steuerinformationen, welche derart ausgestaltet sind, dass sie bei Verwendung des Datenträgers in einer Steuerungseinheit einer Patientenlagerungsvorrichtung das Verfahren zu einer Steuerung einer Fahreinheit einer Patientenlagerungsvorrichtung nach einem der Ansprüche 8 bis 17 durchführen.

## Claims

1. Patient support apparatus (10) with a couch (12), a travel unit (14) which is designed to move the patient support apparatus, a control unit (19) and a sensor unit (21), wherein the sensor unit records at least one environmental parameter of the patient support apparatus and the control unit ascertains a control parameter for controlling the travel unit depending on the at least one environmental parameter, wherein the sensor unit has at least one environmental sensor, which comprises a B₀ field sensor, and wherein the patient support apparatus has a front area with a docking unit for docking with a magnetic resonance apparatus, wherein the sensor unit is arranged in the front area.

2. Patient support apparatus according to claim 1,
**characterised in that** the at least one B₀ field sensor comprises a Hall effect sensor.

3. Patient support apparatus according to one of the preceding claims,
**characterised in that** the sensor unit has at least two environmental sensors which are arranged at a distance from one another.

4. Patient support apparatus according to one of the preceding claims,
**characterised in that** the sensor unit has at least one environmental sensor which is arranged inside the couch.

5. Patient support apparatus according to one of the preceding claims,
**characterised by** a user interface which is designed to adjust a threshold value of the environmental parameter, wherein the threshold value specifies a criterion for the environmental parameter for determining the control parameter for controlling the travel unit by means of the control unit.

6. Patient support apparatus according to one of the preceding claims,
**characterised in that** the control unit has a communication interface for communication with a magnetic resonance apparatus.

7. Magnetic resonance apparatus with a patient support apparatus according to one of claims 1 to 6.

8. Method for controlling a travel unit of a patient support apparatus by means of a control unit during a docking procedure of the patient support apparatus with a magnetic resonance apparatus, comprising the following steps:
- Recording of at least one environmental parameter, wherein the environmental parameter comprises a value of a B₀ field, of the patient support apparatus by means of a sensor unit, wherein the sensor unit has at least one environmental sensor, which comprises a B₀ field sensor,
- Determination of at least one control parameter for activation of the travel unit by means of the control unit, wherein the at least one control parameter is determined depending on the at least one environmental parameter, wherein with the aid of the environmental parameter and with the aid of at least one field map a position of the patient support apparatus is determined in relation to the magnetic resonance apparatus by means of the control unit and
- Activation of the travel unit according to the ascertained control parameter by means of the control unit.

9. Method according to claim 8,
**characterised in that** the environmental parameter comprises a value of a gradient field and/or a value of a B₁ field and/or a number of light markings at the location of the patient support apparatus.

10. Method according to one of claims 8 to 9,
**characterised in that** the travel unit is controlled by means of the control unit depending on the at least one field map.

11. Method according to one of claims 8 to 10,
**characterised in that** a threshold value of the environmental parameter is adjustable, wherein the threshold value specifies a criterion for the environmental parameter for determining the control parameter for controlling the travel unit by means of the control unit.

12. Method according to one of claims 8 to 11,
**characterised in that** the travel unit is automatically controlled with the aid of the ascertained control parameter as soon as a control parameter is ascertained.

13. Method according to one of claims 8 to 12,
**characterised in that** the travel unit is automatically controlled by the control unit with the aid of the ascertained control parameter depending on a user action.

14. Method according to one of claims 8 to 13,
**characterised in that** a height of the couch is determined by means of the control unit with the aid of the environmental parameter.

15. Method according to one of claims 8 to 14,
**characterised in that** with the aid of the environmental parameter a type of magnet of the magnetic resonance apparatus is determined by means of the control unit.

16. Method according to claim 15,
**characterised in that** to determine the type of magnet the environmental parameter is compared with the field maps deposited in the control unit.

17. Method according to one of claims 8 to 16,
**characterised in that** with the aid of the environmental parameter a direction of movement of the patient support apparatus is determined by means of the control unit.

18. Computer program product which comprises a program and can be directly loaded into a storage facility of a programmable control unit of a patient support apparatus, with program means to perform a method for controlling a travel unit of a patient support apparatus according to one of claims 8 to 17, if the program is performed in the control unit of the patient support apparatus.

19. Electronically readable data carrier with electronically readable control information stored thereon which is designed such that it performs the method for controlling a travel unit of a patient support apparatus according to one of claims 8 to 17 when using the data carrier in a control unit of a patient support apparatus.

## Revendications

1. Dispositif (10) de mise en position d'un patient, comprenant une couchette (12), une unité (14) de déplacement, conçue pour déplacer le dispositif de mise en position du patient, une unité (19) de commande et une unité (21) de capteur,
dans lequel l'unité de capteur détecte au moins un paramètre de champ périphérique du dispositif de mise en position d'un patient et l'unité de commande détermine, en fonction du au moins un paramètre de champ périphérique, un paramètre de commande pour commander l'unité de déplacement, l'unité de capteur ayant au moins un capteur de champ périphérique, qui comprend un capteur de champ B0 et le dispositif de mise en position d'un patient ayant une partie avant ayant un dispositif d'amarrage pour s'amarrer à un dispositif à résonnance magnétique, l'unité de capteur étant disposée dans la partie avant.

2. Dispositif de mise en position d'un patient suivant la revendication 1,
**caractérisé en ce que** le au moins un capteur de champ B₀ comprend un capteur de Hall.

3. Dispositif de mise en position d'un patient suivant l'une des revendications précédentes,
**caractérisé en ce que** l'unité de capteur a au moins deux capteurs de champ périphérique, qui sont disposés à distance les uns des autres.

4. Dispositif de mise en position d'un patient suivant l'une des revendications précédentes,
**caractérisé en ce que** l'unité de capteur a au moins un capteur de champ périphérique, qui est disposé à l'intérieur de la couchette.

5. Dispositif de mise en position d'un patient suivant l'une des revendications précédents,
**caractérisé par** une interface d'utilisateur conçue pour régler une valeur de seuil du paramètre de champ périphérique, la valeur de seuil fixant un critère pour le paramètre de champ périphérique, afin de déterminer le paramètre de commande pour commander l'unité de déplacement au moyen de l'unité de commande.

6. Dispositif de mise en position d'un patient suivant l'une des revendications précédentes,
**caractérisé en ce que** l'unité de commande a une interface de communication pour communiquer avec un dispositif à résonnance magnétique.

7. Dispositif à résonnance magnétique ayant un dispositif de mise en position d'un patient suivant l'une des revendications 1 à 6.

8. Procédé de commande d'une unité de déplacement d'un dispositif de mise en position d'un patient au moyen d'une unité de commande pendant une opération d'amarrage du dispositif de mise en position d'un patient à un dispositif à résonnance magnétique, comprenant les stades suivants :
- détection d'au moins un paramètre de champ périphérique, le paramètre de champ périphérique comprenant une valeur d'un champ B₀ du dispositif de mise en position d'un patient, au moyen d'une unité de capteur, l'unité de capteur ayant au moins un capteur de champ périphérique, qui comprend un capteur de champ B₀,
- détermination d'au moins un paramètre de commande pour commander l'unité de déplacement au moyen de l'unité de commande, la détermination du au moins un paramètre de commande s'effectuant en fonction du au moins un paramètre de champ périphérique, dans lequel, à l'aide du paramètre de champ périphérique et à l'aide d'au moins une carte de champ, on détermine une position du dispositif de mise en position du patient par rapport au dispositif à résonnance magnétique, au moyen de l'unité de commande, et
- commande, au moyen de l'unité de commande, de l'unité de déplacement suivant le paramètre de commande déterminé.

9. Procédé suivant la revendication 8,
**caractérisé en ce que** le paramètre de champ périphérique comprend une valeur d'un champ de gradient et/ou une valeur d'un cham B₁ et/ou un certain nombre de repères lumineux à l'emplacement du dispositif de mise en position d'un patient.

10. Procédé suivant l'une des revendications 8 à 9,
**caractérisé en ce que** la commande de l'unité de déplacement s'effectue au moyen de l'unité de commande, en fonction de la au moins une carte de champ.

11. Procédé suivant l'une des revendications 8 à 10,
**caractérisé en ce qu'**une valeur de seuil du paramètre de champ périphérique est réglable, la valeur de seuil fixant un critère au paramètre de champ périphérique, pour déterminer le paramètre de commande, afin de commander l'unité de déplacement, au moyen de l'unité de commande.

12. Procédé suivant l'une des revendications 8 à 11,
**caractérisé en ce que** la commande de l'unité de déplacement s'effectue à l'aide du paramètre de commande déterminé automatiquement par l'unité de commande, dès qu'un paramètre de commande est déterminé.

13. Procédé suivant l'une des revendications 8 à 12,
**caractérisé en ce que** la commande de l'unité de déplacement s'effectue à l'aide du paramètre de commande déterminé automatiquement par l'unité de commande, en fonction d'une action de l'utilisateur.

14. Procédé suivant l'une des revendications 8 à 13,
**caractérisé en ce que** l'on détermine, à l'aide du paramètre de champ périphérique, un niveau de la couchette, au moyen de l'unité de commande.

15. Procédé suivant l'une des revendications 8 à 14,
**caractérisé en ce que** l'on détermine, à l'aide du paramètre de champ périphérique, un type de construction d'un aimant du dispositif à résonnance magnétique, au moyen de l'unité de commande.

16. Procédé suivant la revendication 15,
**caractérisé en ce que**, pour déterminer le type de construction de l'aimant, on compare le paramètre de champ périphérique à des cartes de champ mémorisées dans l'unité de commande.

17. Procédé suivant l'une des revendications 8 à 16,
**caractérisé en ce qu'**à l'aide du paramètre de champ périphérique, on détermine, au moyen de l'unité de commande, une direction de déplacement du dispositif de mise en position d'un patient.

18. Produit de programme d'ordinateur, qui comprend un programme et qui peut être chargé directement dans une mémoire d'une unité de commande programmable d'un dispositif de mise en position d'un patient, comprenant des moyens de programme pour exécuter un procédé de commande de l'unité de déplacement d'un dispositif de mise en position d'un patient suivant l'une des revendications 8 à 17, lorsque le programme est réalisé dans l'unité de commande du dispositif de mise en position d'un patient.

19. Support de données déchiffrables électroniquement, dans lequel de informations de commande déchiffrables électroniquement sont mémorisées, lesquelles informations sont conformées de manière à ce que, lors de l'utilisation du support de données dans une unité de commande d'un dispositif de mise en position d'un patient, elles effectuent le procédé de commande d'une unité de déplacement d'un dispositif de mise en position d'un patient suivant l'une des revendications 8 à 17.
